# FASCICULE DE BREVET EUROPEEN

(11) **EP 0 692 242 B1**
(45) Date de publication et mention de la délivrance du brevet: **09.04.1997**
(21) Numéro de dépôt: 95401568.1
(22) Date de dépôt: 29.06.1995
(51) Int. Cl.: A61K 7/00

(54) **Utilisation de particules creuses déformables dans une composition cosmétique et/ou dermatologique, contenant des matières grasses**
Verwendung von verformbaren, hohlen Teilchen in einer kosmetischen und/oder dermatologischen Zusammensetzung die fette Stoffe enthält
Use of deformable hollow particles in a cosmetic and/or dermatological composition containing fatty substance

(30) Priorité: 11.07.1994 FR 9408569
(43) Date de publication de la demande: 17.01.1996
(73) Titulaire: CENTRE INTERNATIONAL DE RECHERCHES DERMATOLOGIQUES GALDERMA, ( CIRD GALDERMA), 06560 Valbonne (FR)
(72) Inventeur: Allec, Josiane, Les Vergères de Val Constance, F-06600 Antibes (FR); Ferrara, Eve, Le Vendôme, F-06110 Le Cannet (FR); Bara, Isabelle, F-75013 Paris (FR)
(74) Mandataire: Lhoste, Catherine

(56) Documents cités:
- EP-A- 0 566 442
- EP-A- 0 605 284
- DE-A- 2 521 003
- PATENT ABSTRACTS OF JAPAN vol. 10, no. 31 (C-327) (2088) & JP-A-60 184 004 (POLA KASEI K.K.K.)

## Description

La présente invention a pour objet l'utilisation de particules creuses déformables dans une composition cosmétique et/ou dermatologique, contenant des matières grasses. Elle s'applique à toute composition contenant des matières grasses, c'est-à-dire contenant des composés lipophiles comme les huiles et/ou les composés cireux, telle que les émulsions eau-dans-huile ou huile-dans-eau, les gels lipophiles, les pâtes ou produits coulés anhydres.

Ces compositions peuvent en particulier être utilisées pour le maquillage, le soin, l'hygiène de la peau, aussi bien du visage que du corps humain y compris le cuir chevelu et les muqueuses, le soin des cheveux et enfin le traitement thérapeutique de la peau et des muqueuses. Ainsi, la composition à laquelle s'applique l'invention peut être une crème de soin, un baume, un blush ou un fond de teint fluide ou coulé, un onguent dermopharmaceutique, un lait nettoyant ou démaquillant, une composition déodorante ou solaire.

Les compositions contenant une forte teneur en huile, et plus spécialement celles à phase grasse continue, ont tendance à laisser sur la peau un film au toucher gras, collant ou poisseux, s'accompagnant souvent d'un aspect brillant, les rendant peu attrayantes, voire d'utilisation rédhibitoire, surtout lorsqu'il s'agit d'applications répétées sur le cuir chevelu ou le visage, et plus spécialement pour les utilisateurs ou les patients à peau mixte, grasse ou à peau acnéïque. De plus, la présence d'une grande quantité de corps gras, rend difficile l'étalement de la composition sur la peau.

Or, un grand nombre d'actifs cosmétiques et/ou dermatologiques ne peut être formulé qu'en présence d'une grande quantité de corps gras et notamment dans un milieu anhydre ou à phase grasse continue ; ceci est notamment le cas des actifs instables dans l'eau. En outre, certaines compositions destinées aux peaux sèches, voire très sèches, nécessitent la présence d'une phase grasse continue.

Il subsiste donc le besoin d'une composition cosmétique et/ou dermatologique, contenant des matières grasses et susceptible de contenir des actifs lipophiles, ne conférant pas de sensation de gras et/ou de collant sur la peau ni d'aspect brillant.

De façon surprenante, la demanderesse a trouvé qu'il était possible de diminuer le toucher gras et/ou collant, sur la peau d'une composition riche en matières grasses, en introduisant des particules creuses déformables de masse volumique et de granulométrie particulières.

En plus de l'élimination de ces touchers gras et/ou collant, ces particules confèrent de la douceur et de l'homogénéité à l'application (ce qui est très important pour un produit de maquillage), ainsi qu'une plus grande facilité d'étalement.

De façon plus précise, l'invention se rapporte à l'utilisation de particules creuses déformables, dans une composition cosmétique contenant des matières grasses, pour éliminer un toucher collant et/ou gras de la composition dû à ces matières grasses, ces particules ayant une granulométrie allant de 1 µm à 250 µm et étant formées d'un copolymère de chlorure de vinylidène, d'acrylonitrile et de (méth)acrylate de méthyle.

L'invention a aussi pour objet l'utilisation de particules creuses déformables, pour la fabrication d'une composition dermatologique contenant des matières grasses, pour éliminer un toucher collant et/ou gras de la composition dû à ces matières grasses, ces particules ayant une granulométrie allant de 1 µm à 250 µm et étant formées d'un copolymère de chlorure de vinylidène, d'acrylonitrile et de (méth)acrylate de méthyle.

Par" déformable", il faut comprendre que les particules sont souples et élastiques : après écrasement, elles reprennent leur forme initiale.

Certes, il est connu de la demande de brevet français n° 93-00990 d'utiliser des particules creuses dans une composition cosmétique ou dermopharmaceutique pour les rendre moins collantes, onctueuses et douces, mais ces compositions sont des gels aqueux exempts de matières grasses. En outre, ce document suggère d'éliminer les matières grasses et donc l'origine du toucher gras et/ou collant.

Par ailleurs, le document EP-A-605284 décrit un procédé d'extrusion permettant d'obtenir une composition de maquillage contenant une quantité importante de poudre lègère.

Le document JP-A-60184004 décrit une préparation cosmétique contenant des particules creuses, ayant la propriété d'être douce à l'application et efficace pour contrôler le sébum et l'hydratation de la peau.

Le document DE-A-2521003 enseigne que l'incorporation de microsphères creuses permet d'obtenir des produits ayant une consistance de mousse.

Le document EP-A-566442 se rapporte à une composition de rouge à lèvres dont l'effet matifiant est obtenu grâce à l'incorporation de microsphères creuses.

Selon l'invention, il est possible de réaliser une composition quasi-anhydre contenant moins de 10 % d'eau par rapport au poids total de la composition. Avec ce faible pourcentage d'eau, il est tout-à-fait surprenant de ne ressentir aucun toucher gras ni collant sur la peau. Par ailleurs, la composition de l'invention permet de traiter cosmétiquement et/ou dermatologiquement la peau, selon le ou les actifs utilisés, tout en l'hydratant, la nourrissant et/ou la protégeant.

De façon avantageuse, les particules ont une granulométrie inférieure à 100 µm. En effet, plus les particules sont fines, plus la composition est douce à l'application. De préférence, les particules ont une granulométrie allant de 10 µm à 50 µm.

De façon avantageuse, les particules ont une masse volumique allant de 15 kg/m³ à 200 kg/m³ et mieux supérieure à 40 kg/m³ et/ou inférieure à 100 kg/m³ et notamment allant de 60 kg/m³ à 80 kg/m³.

On peut, par exemple, utiliser un copolymère contenant : de 1 % à 60 % de motifs dérivés du chlorure de vinylidène, de 20 % à 90 % de motifs dérivés d'acrylonitrile et de 1 % à 50 % de motifs dérivés d'un monomère acrylique, la somme des pourcentages (en poids) étant égale à 100. Le monomère acrylique est par exemple un acrylate ou méthacrylate de méthyle et, en particulier le méthacrylate. Ces particules se présentent notamment à l'état sec ou hydraté.

Ce copolymère est non toxique et non irritant pour la peau.

De préférence, les particules se présentent sous forme de microsphères.

Les particules de l'invention peuvent être obtenues, par exemple, selon les procédés des brevets et demandes de brevet EP-56 219, EP-348 372, EP-486 080, EP-320 473, EP-112 807 et US-3 615 972.

La cavité interne des particules contient en principe un gaz qui peut être de l'air, de l'azote ou un hydrocarbure comme l'isobutane ou l'isopentane.

Les particules creuses utilisables dans l'invention sont en particulier celles ayant une granulométrie de 18 µm et une masse volumique de 60 kg/m³ à 80 kg/m³ appelées ci-dessous EL 23. Ces particules confèrent le maximum de qualités cosmétiques. En outre, elles ont l'avantage d'être incorporables dans des formulations de viscosité assez faible (de l'ordre de 20 ps soit 2 mPa.s) sans procurer de "crémage" des particules à la surface du milieu à l'inverse de particules de densité plus faible.

Comme autres particules utilisables dans l'invention, on peut citer celles vendues sous la marque EXPANCEL par la Société Nobel Casco telles que l'EXPANCEL 551 DE 20 de granulométrie de 30 µm et de masse volumique de 65 kg/m³ environ ; l'EXPANCEL 551 DE 50 de granulométrie de 40 µm.

La majeure partie des propriétés des particules de l'invention est due à leur déformabilité.

Dans les compositions de l'invention, on utilise de préférence de 0,1 % à 10 % en poids de particules et mieux de 0,5 % à 5 % en poids et encore de 0,5 % à 2 %, par rapport au poids total de la composition.

Par ailleurs, ces particules ont un pouvoir opacifiant des gels anhydres et des émulsions à phase continue huileuse, translucides, conférant un aspect crémeux, brillant, blanc ou ivoire, à la composition, augmentant ainsi son attrait pour le consommateur. En particulier, la demanderesse a pu constater que le pouvoir opacifiant des particules creuses selon l'invention était supérieur à celui de plusieurs autres types de particules, testés. Ainsi, il est possible d'obtenir une crème blanche avec 1 % en poids de particules creuses selon l'invention alors qu'il faut au moins 2 % en poids de particules pour les autres types de particules. En particulier, même avec 4 % en poids de MICROPEARL M305 de chez la Société SEPPIC, qui sont des particules sphériques pleines de polyméthacrylate de méthyle réticulé de 5 µm à 20 µm, on obtient un gel grisâtre non acceptable d'un point de vue aspect visuel.

Les particules creuses déformables thermoplastiques peuvent être introduites dans des compositions du type émulsions eau-dans-huile, eau-dans-silicone, huile-dans-eau, des compositions anhydres, des gels lipophiles ou encore des solutions huileuses. Ces compositions sont semi-solides ou solides.

Les huiles, les composés cireux, les gélifiants et les tensioactifs nécessaires à la formulation et à la stabilisation de ces compositions sont ceux classiquement utilisés dans le domaine cosmétique et/ou dermatologique.

Les huiles et/ou composés cireux représentent de 0,5 % à 99,9 % du poids total de la composition, de préférence de 1 % à 80 % et mieux de 1 % à 40 %. La quantité d'huile et/ou de cire dépend de la forme galénique de la composition.

Comme huiles, on peut citer les huiles minérales (vaseline) ; végétales (huile d'amande douce, de macadamia, de pépin de cassis) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés ; siliconées (cyclométhicone, polydiméthysiloxanes ou PDMS) ou fluorées.

Comme composés cireux, on peut citer l'huile de jojoba, la paraffine, la cire de carnauba, la cire d'abeille.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

Comme tensioactifs (émulsionnants et coémulsionnants), on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols ainsi que les tensioactifs anioniques (alkylphosphate de K ou de Na).

Les compositions de l'invention peuvent aussi contenir divers ingrédients classiquement utilisés dans le domaine cosmétique, dermatologique ou dermopharmaceutique comme les matières colorantes (pigments, colorants), les solvants, les conservateurs, les parfums, les actifs hydratants, les agents absorbant les rayons ultraviolets (filtres solaires, pigments), les agents pulvérulents autres que les particules creuses déformables, des agents bactéricides, antitranspirants et/ou des absorbeurs d'odeur. Les pigments sont en particulier les oxydes de fer, de titane, de zinc.

Ces compositions peuvent, en outre, contenir un ou plusieurs actifs cosmétiques ou dermatologiques hydrophiles et mieux lipophiles, notamment en vue de traiter et/ou prévenir les affections cutanées telles que l'acné, les mycoses, I'eczéma, la rosacée, les dermites séborrhéïques, les hélio dermatoses, le vieillissement cutané ainsi que les affections du cuir chevelu. Ces compositions sont destinées au traitement de la peau par voie topique.

La composition de l'invention contient avantageusement un ou plusieurs actifs cosmétiques et/ou dermatologiques. Parmi ces actifs, on peut citer à titre d'exemple :
- les agents modulant la différenciation et/ou la prolifération et/ou la pigmentation cutanée tels que l'acide rétinoïque et ses isomères ; le rétinol et ses esters ainsi que des composés de synthèse comme l'acide 6-[3-(-adamantyl)-4-méthoxyphényl]-2-napthoïque ; la vitamine D et ses dérivés ; les oestrogènes tels que l'estradiol ; l'acide kojique ou l'hydroquinone ;
- les antibactériens tels que le phosphate de clindamycine, I'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, les dérivés d'acide gras polyinsaturés comme les amides de l'acide 5, 8, 11-eicosatriénoïque, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement l'acide salicylique et ses dérivés comme l'acide n-octanoyl-5-salicylique, les α-hydroxyacides tels que l'acide glycolique, l'acide lactique, l'acide citrique et de manière générale les acides de fruits ;
- les agents anti-radicaux libres, tels que l'alpha-tocophérol ou ses esters, les superoxyde dismutases, certains chélatants de métaux ou l'acide ascorbique et ses esters ;
- les antiséborrhéïques tels que la progestérone ;
- les antipelliculaires comme l'octopirox ou les sels de pyrithione, notamment de zinc ou de sodium de zinc ;
- les antiacnéïques comme l'acide rétinoïque ou le peroxyde de benzoyle ;
- les vitamines (F) ;
- les anthranoïdes comme la 1, 8, 10-tripropionyl-9-anthrone ;
- les cicatrisants comme la vitamine C ;
- les absorbeurs d'odeurs.

La concentration en actif dans la composition dépend de la nature de l'actif ; elle est généralement choisie de 0,0001 % à 20 % en poids par rapport au poids total de la composition.

Ces actifs peuvent être utilisés purs ou encapsulés dans des sphérules comme les micro- ou nanosphères, les micro- ou nanocapsules, les micro- ou nanoéponges, les vésicules lipidiques contenant des lipides ioniques et/ou non-ioniques connus sous le nom de liposomes. Les procédés d'encapsulation de l'invention sont aux classiques utilisés dans le domaine cosmétique et/ou dermatologique et notamment décrit dans les documents FR-A-2315991 et FR-A-2485921.

D'autres caractéristiques et avantages de l'invention ressortiront mieux de la description qui va suivre, donnée à titre illustratif et non limitatif.

La demanderesse a testé plusieurs charges de natures différentes dans un même milieu anhydre en vue de comparer leur influence sur la texture et l'opacification du milieu. Certaines ont été utilisées à la concentration minimale pour obtenir l'effet opacifiant. Pour les charges ne donnant pas de résultat satisfaisant, leur concentration à été augmentée progressivement jusqu'à 6 % en poids. La composition du milieu anhydre, en poids, était la suivante :

| | |
|---|---|
| -Perhydrosqualène | 70 % |
| -Silicone fluid 245 de chez Dow Corning | 20 % |
| -Aerosil R972 de chez Degussa ** | 8 % |

| | |
|---|---|
| (**) Silice pyrogénée hydrophobe, traitée par tri-méthylsiloxane. | |

Les résultats obtenus sont donnés dans le tableau 1 ci-après. On constate, d'après ce tableau, que seules les particules d'Expancel assurent un toucher non gras, non collant au gel et lui confèrent l'aspect d'une crème, sans relarguage d'huile, et donc lui confèrent une bonne stabilité.

**Tableau 1**

| **Type de charge** | **Pourcentage utilisé** | **Résultats** | |
|---|---|---|---|
| | | **Aspect** | **Toucher** |
| Micropearl M100 Poudre sphérique de polyméthacrylate de 8 µm à 10 µm densité : 1,2 | 2 % gel encore translucide et non matifié. | Gel opalescent présentant un relarguage huileux en surface. | cireux à l'application, surtout vers la fin. Pénètre lentement. |
| | 6 % commence à s'opacifier. | | |
| Luzenac 15 Mod (talc) | 4 % | Gel grisâtre et opalescent. | Gras. Ne pénètre pas. Accroche à l'application. |
| Orgasol 2002 UD NAT COS Microéponges de Nylon de 2 µm à 10 µm | 3 % | Gel crème blanc, présentant un relarguage huileux | Glissant, ne pénètre pas très bien. |
| | | | Toucher doux. |
| TiO₂ U.S.P. BC Dioxyde de titane micronisé (Whittaker Clark) | 3 % | Crème blanche, brillante mais présence de grains. | Gras, pénètre rapidement et toucher collant qui accroche à l'application |
| Particules El 23 | 1 % | Crème ivoire, brillante. | Glissant. Pénétration lente. Toucher non gras. |
| Expancel 551 DE 20 | 1 % | Crème brillante, blanc cassé. | Pénétration moyenne. Glissant Toucher final doux, non collant, soyeux, poudreux. |
| Polytrap 6603 Polyméthylméthacrylate, aggrégats de 200 µm à 1200 µm. Densité : 0,06 | 4 % | Gel opalescent et granuleux. | On ne sent pas les grains. Gras à l'application. Ne pénètre pas. Toucher cireux. |
| Oxyde de zinc U.S.P. (Whittaker Clark) | 4 % | Crème blanche brillante. | Gras. On voit des grains mais on ne les sent pas. |

On donne ci-après différents exemples de compositions conformes à l'invention.

### Exemple 1 : Gel anhydre dermopharmaceutique pour le traitement de l'acné

| | |
|---|---|
| - Clindamycine phosphate | 1,2 % |
| - Perhydrosqualène | qs 100 % |
| - Silicone fluide (245 de Wacker Silicones) | 20 % |
| - Aérosil R 972 | 8 % |
| - Particules El 23 | 1 % |

Le gel obtenu est crémeux, lisse, brillant, très doux, sans toucher gras ni collant. Il peut être utilisé pour toute type de peau à tendance acnéïque.

### Exemple 2 : Gel anhydre dermopharmaceutique pour le traitement de l'acné pour être spécifiquement délivré au niveau des follicules

| | |
|---|---|
| - Microshères d'Orgasol contenant 4,3 % d'acide 6-[3(1-adamantyl)-4-méthoxyphényl]-2-naphtoïque | 2,5 % |
| - Perhydrosqualène | qs 100 % |
| - Silicone fluide 245 | 20 % |
| - Aérosil R 972 | 8 % |
| - Particules El 23 | 1 % |

Les propriétés physiques du gel sont les mêmes que celles de l'exemple 1.

### Exemple 3 : Emulsion E/H pour le traitement des peaux sèches, ou des xéroses

| *Phase A : grasse* | |
|---|---|
| - Abil EM 90 de Goldschmidt (cétyldiméthicone copolyol) | 2,5 % |
| - DC 344 Fluid de Dow Corning (cyclométhicone) | 15 % |
| - DC 593 Fluid de Dow Corning (cyclométhicone) | 3,5 % |
| - Witconol APM de Witco (Polypropylèneglycol myristyl éther à 3 moles de propylène glycol) | 6 % |

| *Phase B : aqueuse* | |
|---|---|
| - Glycérine | 3 % |
| - Acide lactique | 5 % |
| - NH₃ (solution à 32 %) | qsp pH = 4 |
| - NaCl | 0,6 % |
| - Conservateur | 0,15 % |
| - Eau | qsp 100 % |

| *Phase C :* | |
|---|---|
| - Particules El 23 | 1 % |

**Mode opératoire** : On effectue le mélange des composés de la phase grasse. L'émulsion se fait à froid avec une hélice typhon en ajoutant à la phase grasse la phase aqueuse à la pipette. Puis on ajoute la phase C et on mélange avec la même hélice, pendant 5/10 minutes.

### Exemple 4 : Fond de teint crémeux anhydre

| | |
|---|---|
| - Gel de Miglyol * | 40 % |
| - Pentadiméthylsiloxane | 29 % |
| - Triglycérides d'acides caprylique-caprique | 14,4 % |
| - Oxyde de fer | 1,05 % |
| - Dioxyde de titane | 4,35 % |
| - Particules El 23 | 0,5 % |
| - Aérosil R972 | 0,7 % |
| - Talc (silicate de magnésium) | 6 % |
| - Amidon | 4 % |
| - Conservateur | qs |

| | |
|---|---|
| (*) Hectorite modifiée par du chlorure de stéaryl di-méthyl benzyl ammonium dans di-caprylate/di-caprate de glycéryle et carbonate de propylène. | |

On obtient un fond de teint anhydre crémeux, très doux, avec un toucher non gras et non collant, malgré la forte concentration en corps gras.

### Exemple 5 : Crème déodorante aqueuse (huile/eau) (nomenclature CTFA)

| *Phase A : grasse* | |
|---|---|
| - Cetearyl alcohol (corps gras, gélifiant) | 4 % |
| - Glyceryl stearate (corps gras, gélifiant) | 2,5 % |
| - Steareth-25 (émulsionnant) | 1,05 % |
| - Stearyl alcohol (corps gras, gélifiant) | 1,05 % |
| - Ceteareth-33 (émulsionnant) | 1 % |
| - Dimethicone | 1 % |
| - Ceteth-20 (émulsionnant) | 0,4 % |
| - Conservateur | qs |

| *Phase B : aqueuse* | |
|---|---|
| - Eau | qsp 100 % |
| - Aluminium chlorohydrate (antitranspirant) | 13 % |
| - Conservateur | qs |

| *Phase C : actif* | |
|---|---|
| - Parfum | qs |
| - Bactéricide | qs |

| *Phase D :* | |
|---|---|
| - EXPANCEL 551 DE 50 | 1 % |

**Mode opératoire :** Chauffer la phase B à 70-80 °C, sous agitation. Chauffer la phase A à 70-80 °C. Lorsque les deux phases sont bien homogènes, incorporer A dans B, sous agitation forte pendant 10 min. Refroidir sous agitation plus faible. A 45 °C, ajouter la phase C prémélangée. Agiter fortement pendant 5 min. Refroidir sous agitation plus faible. Vers 30 °C, incorporer lentement la phase D. Poursuivre le refroidissement jusqu'à la température ambiante.

On obtient une crème souple et douce, à effet poudré à l'étalement.

### Exemple 6 : Crème anhydre déodorante (nomenclature CTFA)

| *Phase A :* | |
|---|---|
| - Stearalkonium hectorite (gélifiant) | 4,2 % |
| - Propylene carbonate (agent de mise en suspension) | 1,4 % |
| - Caprylic/Capric Triglycerides | 29,4 % |
| - Dimethicone | 5 % |
| - Isohexadecane (solvant) | qsp 100 % |

| *Phase B :* | |
|---|---|
| - Chlorohydrate aluminium (antitranspirant) | 15 % |
| - EXPANCEL 551 DE 50 | 1 % |

| *Phase C :* | |
|---|---|
| - Parfum | qs |

| *Phase D :* | |
|---|---|
| - Talc | 6 % |

**Mode opératoire :** Mélanger les composés de la phase A à température ambiante. Incorporer ensuite la phase C, toujours à température ambiante. Lorsque tout est homogène, incorporer B et D lentement.

La crème obtenue est particulièrement douce, non grasse, non collante, et procure par son impact sec, une sensation de plus grande sécurité contre l'humidité axillaire.

### Exemple 7 : Aérosol (nomenclature CTFA)

| *Phase A :* | |
|---|---|
| - Cyclomethicone | qsp 100 % |
| - Quaternium-18 bentonite | 3 % |
| - Triethylcitrate | 7 % |
| - Dimethiconol | 1,4 % |
| - Isopropyl palmitate | 6 % |

| *Phase B :* | |
|---|---|
| - Aluminium chlorohydrate | 30 % |

| *Phase C :* | |
|---|---|
| - EXPANCEL 551 DE 50 | 1,5 % |

**Mode opératoire :** Mélanger les ingrédients de la phase A à froid. Ajouter lentement le chlorohydrate d'aluminium, puis l'EXPANCEL. Mélanger vigoureusement pour obtenir une suspension homogène. Cette dernière peut ensuite être pressurisée de la façon suivante : 13 % de jus, 87 % de gaz comprimé ou liquéfié.

Le jus délivré laisse un impact doux, poudré sur la peau.

## Revendications

1. Utilisation de particules creuses déformables, dans une composition cosmétique contenant des matières grasses, pour éliminer un toucher collant et/ou gras de la composition dû à ces matières grasses, ces particules présentant une granulométrie allant de 1 µm à 250 µm, et étant formées d'un copolymère de chlorure de vinylidène, d'acrylonitrile et de (méth)acrylate de méthyle, expansé.

2. Utilisation de particules creuses déformables, pour la fabrication d'une composition dermatologique contenant des matières grasses, pour éliminer un toucher collant et/ou gras de la composition dû à ces matières grasses, ces particules présentant une granulométrie allant de 1 µm à 250 µm, et étant formées d'un copolymère de chlorure de vinylidène, d'acrylonitrile et de (méth)acrylate de méthyle, expansé.

3. Utilisation selon la revendication 1 ou 2, caractérisée en ce que les particules ont une granulométrie inférieure à 100 µm.

4. Utilisation selon la revendication précédente, caractérisée en ce que les particules ont une granulométrie allant de 10 µm à 50 µm.

5. Utilisation selon l'une des revendications précédentes, caractérisée en ce que les particules ont une masse volumique allant de 15 kg/m³ à 200 kg/m³.

6. Utilisation selon l'une des revendications précédentes, caractérisée en ce que les particules ont une masse volumique allant de 40 kg/m³ à 100 kg/m³.

7. Utilisation selon l'une des revendications précédentes, caractérisée en ce que les particules représentent de 0,01 % à 10 % du poids total de la composition.

8. Utilisation selon l'une des revendications précédentes, caractérisée en ce que les matières grasses représentent de 0,5 % à 99 % du poids total de la composition.

9. Utilisation selon l'une des revendications précédentes, caractérisée en ce qu'elle contient, en outre, au moins un adjuvant choisi parmi les gélifiants, les tensioactifs, les actifs hydrophiles, les actifs lipophiles, les parfums, les conservateurs, les solvants, les matières colorantes, les argiles, les agents de texture.

10. Utilisation selon lune des revendications précédentes, caractérisée en ce qu'elle contient des pigments

11. Utilisation selon l'une des revendications précédentes, caractérisée en ce qu'elle se présente sous forme d'une émulsion eau-dans-huile, d'un gel lipophile, d'une composition anhydre.

12. Utilisation selon l'une des revendications précédentes, caractérisée en ce qu'elle contient, en outre, au moins un actif lipophile.

13. Utilisation selon l'une des revendications précédentes, caractérisée en ce qu'elle contient, en outre, un actif choisi parmi les agents modulant la différenciation et/ou la prolifération et/ou la pigmentation cutanée, les agents antibactériens, antiparasitaires, antitranspirants, antifongiques, anti inflammatoires, antimycotiques, antiprurigineux, antiviraux, kératolytiques, antiradicaux libres, antiacnéïques, antiséborrhéïques, anesthésiques et cicatrisants. absorbeurs d'odeur.

14. Utilisation selon la revendication 12 ou 13, caractérisée en ce que l'actif est encapsulé.

## Claims

1. Use of deformable hollow particles in a cosmetic composition containing fatty substances, for eliminating a sticky and/or greasy feel of the composition due to these fatty substances, these particles possessing a particle size ranging from 1 µm to 250 µm and being composed of an expanded copolymer of vinylidene chloride, acrylonitrile and methyl (meth)acrylate.

2. Use of deformable hollow particles for the manufacture of a dermatological composition containing fatty substances, for eliminating a sticky and/or greasy feel of the composition due to these fatty substances, these particles possessing a particle size ranging from 1 µm to 250 µm and being composed of an expanded copolymer of vinylidene chloride, acrylonitrile and methyl (meth)acrylate.

3. Use according to Claim 1 or 2, characterized in that the particles have a particle size of less than 100 µm.

4. Use according to the preceding claim, characterized in that the particles have a particle size ranging from 10 µm to 50 µm.

5. Use according to one of the preceding claims, characterized in that the particles have a density ranging from 15 kg/m³ to 200 kg/m³.

6. Use according to one of the preceding claims, characterized in that the particles have a density ranging from 40 kg/m³ to 100 kg/m³.

7. Use according to one of the preceding claims, characterized in that the particles represent from 0.01% to 10% of the total weight of the composition.

8. Use according to one of the preceding claims, characterized in that the fatty substances represent from 0.5% to 99% of the total weight of the composition.

9. Use according to one of the preceding claims, characterized in that it contains, in addition, at least one adjuvant chosen from gelling agents, surfactants, hydrophilic active agents, lipophilic active agents, perfumes, preservatives, solvents, colouring matter, clays and texture agents.

10. Use according to one of the preceding claims, characterized in that it contains pigments.

11. Use according to one of the preceding claims, characterized in that it takes the form of a water-in-oil emulsion, lipophilic gel or anhydrous composition.

12. Use according to one of the preceding claims, characterized in that it contains, in addition, at least one lipophilic active agent.

13. Use according to one of the preceding claims, characterized in that it contains, in addition, an active agent chosen from agents modulating differentiation and/or proliferation and/or skin pigmentation, antibacterial, antiparasitic, antiperspirant, antifungal, anti-inflammatory, antimycotic, antipruritic, antiviral, keratolytic, anti-free-radical, anti-acne, antiseborrhoeic, anaesthetic and cicatrizing agents and odour absorbers.

14. Use according to Claim 12 or 13, characterized in that the active agent is encapsulated.

## Patentansprüche

1. Verwendung von verformbaren Hohlpartikeln in einer kosmetischen Zusammensetzung, die Fettsubstanzen enthält, um ein klebriges und/oder fettiges Anfühlen der Zusammensetzung zu beseitigen, das von den Fettsubstanzen herrührt, wobei die Partikel eine Korngröße von 1 bis 250 µm aufweisen und aus eincm expandierten Copolymer von Vinylidenchlorid, Acrylonitril und Methyl(meth)acrylat gebildet sind.

2. Verwendung von verformbaren Hohlpartikeln zur Herstellung einer dermatologischen Zusammensetzung, die Fettsubstanzen enthält, um ein klebriges und/oder fettiges Anfühlen der Zusammensetzung zu beseitigen, das von den Fettsubstanzen herrührt, wobei die Partikel eine Korngröße von 1 bis 250 µm aufweisen und aus einem expandierten Copolymer von Vinylidenchlorid, Acrylonitril und Methyl(meth)acrylat gebildet sind.

3. Verwendung nach Anspruch 1 oder 2, dadurch gekennzeichnet, daß die Partikel eine Korngröße unter 100 µm aufweisen.

4. Verwendung nach dem vorhergehenden Anspruch, dadurch gekennzeichnet, daß die Partikel eine Korngröße von 10 bis 50 µm aufweisen.

5. Verwendung nach einem der vorhergehenden Ansprüche, dadurch gekennzeichnet, daß die Partikel eine Dichte von 15 bis 200 kg/m³ aufweisen.

6. Verwendung nach einem der vorhergehenden Ansprüche, dadurch gekennzeichnet, daß die Partikel eine Dichte von 40 bis 100 kg/m³ aufweisen.

7. Verwendung nach einem der vorhergehenden Ansprüche, dadurch gekennzeichnet, daß die Partikel 0,01 bis 10 % des Gesamtgewichts der Zusammensetzung ausmachen.

8. Verwendung nach einem der vorhergehenden Ansprüche, dadurch gekennzeichnet, daß die Fettsubstanzen 0,5 bis 99 % des Gesamtgewichte der Zusammensetzung ausmachen.

9. Verwendung nach einem der vorhergehenden Ansprüche, dadurch gekennzeichnet, daß sie ferner mindestens einen Hilfsstoff enthält, der unter den Gelbildnern, grenzflächenaktiven Stoffen, hydrophilen Wirkstoffen, lipophilen Wirkstoffen, Parfums, Konservierungsmitteln, Lösungsmitteln, färbenden Materialien, Tonen und Texturmitteln ausgewählt ist.

10. Verwendung nach einem der vorhergehenden Ansprüche, dadurch gekennzeichnet, daß sie Pigmente enthält.

11. Verwendung nach einem der vorhergehenden Ansprüche, dadurch gekennzeichnet, daß sie in Form einer Wasser-in-Öl-Emulsion, als lipophiles Gel oder als wasserfreie Zusammensetzung vorliegt.

12. Verwendung nach einem der vorhergehenden Ansprüche, dadurch gekennzeichnet, daß sie ferner mindestens einen lipophilen Wirkstoff enthält.

13. Verwendung nach einem der vorhergehenden Ansprüche, dadurch gekennzeichnet, daß sie ferner einen Wirkstoff enthält, der unter den Mitteln ausgewählt ist, die die Differenzierung und/oder Proliferation und/oder Pigmentierung der Haut modulieren, den antibakteriellen Mitteln, den Mitteln gegen Parasiten, den Antitranspirantien, Mitteln gegen Pilze, entzündungshemmenden Mitteln, Antimykotiky, Mitteln gegen Juckreiz, Mitteln gegen Viren, Keratolytika, Mitteln gegen freie Radikale, Mitteln gegen Akne, Mitteln gegen Seborrhoe, Anästhetika, wundheilenden Mitteln und Geruchsabsorbern ausgewählt ist.

14. Verwendung nach einem der Ansprüche 12 oder 13, dadurch gekennzeichnet, daß der Wirkstoff eingekapselt ist.
